# EUROPEAN PATENT APPLICATION

(11) **EP 4 331 598 A1**
(43) Date of publication of application: **06.03.2024**
(21) Application number: 22192650.4
(22) Date of filing: 29.08.2022
(51) Int. Cl.: A61K 36/07, A61P 31/12

(54) **ANTIVIRAL FUNGAL EXTRACTS**

(71) Applicant: Mycotech Pharma AS, 0283 Oslo (NO)
(72) Inventor: SCHMIDT, Ralf, N-1394 Nesbru (NO); FIEBICH, Bernd, D-79111 Freiburg (DE)
(74) Representative: Onsagers AS

(57) **Abstract**

The present disclosure provides compositions able to prevent coronavirus infections. As demonstrated by the examples herein, compositions comprising an extract from Agaricus blazei, Grifola frondosa and Hericium erinaceus may interfere with the interaction between the Spike S1 protein and ACE2. Accordingly, such compositions may provide therapeutic or prophylactic alternatives to the known treatments.

## Description

### Field

The present disclosure concerns mushroom extracts and their use in antiviral drugs or food supplements.

### Background

Coronavirus disease 2019 (COVID-19) increases the risk of developing Acute Respiratory Distress Syndrome (ARDS), which is often fatal at the late stages of the infection when SARS-CoV-2 causes significant damage to the lungs. Several COVID-19 Vaccines have been approved or Authorized for Emergency Use by FDA. They include:
- Comirnaty and Pfizer-BioNTech COVID-19 Vaccine
- Spikevax and Moderna COVID-19 Vaccine
- Janssen COVID-19 Vaccine
- Novavax COVID-19 Vaccine, Adjuvanted
The FDA has approved the antiviral drug Veklury (remdesivir) for adults and certain pediatric patients with COVID-19, but this is an intravenous therapy. The FDA has also approved the immune modulator Olumiant (baricitinib) for certain hospitalized adults with COVID-19.

However, other alternatives would be useful, in particular drugs for oral administration or even non-drug alternatives such as food supplements.

### Summary

The present disclosure provides compositions able to prevent coronavirus infections. As demonstrated by the examples herein, compositions comprising an extract from Agaricus blazei, Grifola frondosa and Hericium erinaceus may interfere with the interaction between the SARS-CoV-2 Spike S1 protein and membrane bound Angiotensin Converting Enzyme 2 (ACE2). Accordingly, such compositions may provide therapeutic or prophylactic alternatives to the known treatments.

In a first embodiment, we provide a pharmaceutically acceptable composition comprising
a. an extract from Agaricus blazei,
b. an extract from Grifola frondosa, and/or
c. an extract from Hericium erinaceus
for use in treatment of coronavirus infections.

In a first aspect of the first embodiment, the composition comprises
an extract from Agaricus blazei,
an extract from Grifola frondosa, and
an extract from Hericium erinaceus.

In a second aspect of the first embodiment, the treatment is therapeutic or prophylactic.

In a third aspect of the first embodiment, the coronavirus is SARS-CoV-2.

In a fourth aspect of the first embodiment, the composition is an aqueous solution.

In a fifth aspect of the first embodiment, the composition is administered orally, intranasally or pulmonary.

In a sixth aspect of the first embodiment, the composition comprises an extract obtained by aqueous extraction of a mixture comprising 50 to 90 wt% mycelium from Agaricus blazei.

In a seventh aspect of the first embodiment, the composition comprises an extract obtained by aqueous extraction of a mixture comprising 10 to 50 wt% mycelium from Grifola frondosa.

In an eighth aspect of the first embodiment, the composition comprises an extract obtained by aqueous extraction from Hericium erinaceus stalks.

In a ninth aspect of the first embodiment, the extracts are obtained by aqueous extraction of mycelium from Agaricus blazei, mycelium from Grifola frondosa and Hericium erinaceus stalks.

In a tenth aspect of the first embodiment, the extract from Agaricus blazei is an aqueous solution comprising 2 g to 3.6 g dry matter material per liter.

In a eleventh aspect of the first embodiment, the extract from Grifola frondosa is an aqueous solution comprising 0.04 g to 0.8 g dry matter material per liter.

In a twelfth aspect of the first embodiment, the extract from Hericium erinaceus is an aqueous solution comprising 0.4 g to 1.2 g dry matter material per liter.

In a thirteenth aspect of the first embodiment, the pH is the range of 5 to 8.

Any one of the aspects of the first embodiment, can be implemented in combination. For example, the first and second aspect may be implemented in combination, the first and third aspect may be implemented in combination, and of course, the first, second and third aspect may be implemented in combination.

In a second embodiment, we provide a method of treatment of a coronavirus infection, comprising the step of administering a pharmaceutically acceptable composition to a subject in need thereof, wherein a pharmaceutically acceptable composition comprises
a. an extract from Agaricus blazei,
b. an extract from Grifola frondosa, and/or
c. an extract from Hericium erinaceus.

In a first aspect of the second embodiment, the composition comprises
an extract from Agaricus blazei,
an extract from Grifola frondosa, and
an extract from Hericium erinaceus.

In a second aspect of the second embodiment, the treatment is therapeutic or prophylactic.

In a third aspect of the second embodiment, the coronavirus is SARS-CoV-2.

In a fourth aspect of the second embodiment, the composition is an aqueous solution.

In a fifth aspect of the second embodiment, the composition is administered orally, intranasally or pulmonary.

In a sixth aspect of the second embodiment, the composition comprises an extract obtained by aqueous extraction of a mixture comprising 50 to 90 wt% mycelium from Agaricus blazei.

In a seventh aspect of the second embodiment, the composition comprises an extract obtained by aqueous extraction of a mixture comprising 10 to 50 wt% mycelium from Grifola frondosa.

In an eighth aspect of the second embodiment, the composition comprises an extract obtained by aqueous extraction from Hericium erinaceus stalks.

In a ninth aspect of the second embodiment, the extracts are obtained by aqueous extraction of mycelium from Agaricus blazei, mycelium from Grifola frondosa and Hericium erinaceus stalks.

In a tenth aspect of the second embodiment, the extract from Agaricus blazei is an aqueous solution comprising 2 g to 3.6 g dry matter material per liter.

In a eleventh aspect of the second embodiment, the extract from Grifola frondosa is an aqueous solution comprising 0.04 g to 0.8 g dry matter material per liter.

In a twelfth aspect of the second embodiment, the extract from Hericium erinaceus is an aqueous solution comprising 0.4 g to 1.2 g dry matter material per liter.

In a thirteenth aspect of the second embodiment, the pH is the range of 5 to 8.

Any one of the aspects of the second embodiment, can be implemented in combination. For example, the first and second aspect may be implemented in combination, the first and third aspect may be implemented in combination, and of course, the first, second and third aspect may be implemented in combination.

In a third embodiment, we provide a method for improving the immune system readiness in a subject, comprising the step of consuming a composition comprising
a. an extract from Agaricus blazei
b. an extract from Grifola frondosa, and/or
c. an extract from Hericium erinaceus.

In a first aspect of the third embodiment, the composition comprises
an extract from Agaricus blazei,
an extract from Grifola frondosa, and
an extract from Hericium erinaceus.

In a second aspect of the third embodiment, the composition comprises an extract obtained by aqueous extraction of a mixture comprising 50 to 90 wt% mycelium from Agaricus blazei.

In a third aspect of the third embodiment, the composition comprises an extract obtained by aqueous extraction of a mixture comprising 10 to 50 wt% mycelium from Grifola frondosa.

In a fourth aspect of the third embodiment, the composition comprises an extract obtained by aqueous extraction from Hericium erinaceus stalks.

In a fifth aspect of the third embodiment, the immune system readiness is improved with respect to coronavirus challenges.

In a sixth aspect of the third embodiment, the composition is an aqueous solution.

In a seventh aspect of the third embodiment, the subject is a healthy human.

In an eighth aspect of the third embodiment, the composition is consumed daily. In an nineth aspect of the third embodiment, the composition is in the form of a food supplement, nutraceutical or functional food.

Any one of the aspects of the third embodiment, can be implemented in combination. For example, the first and second aspect may be implemented in combination, the first and third aspect may be implemented in combination, and of course, the first, second and third aspect may be implemented in combination.

### Brief description of the figures

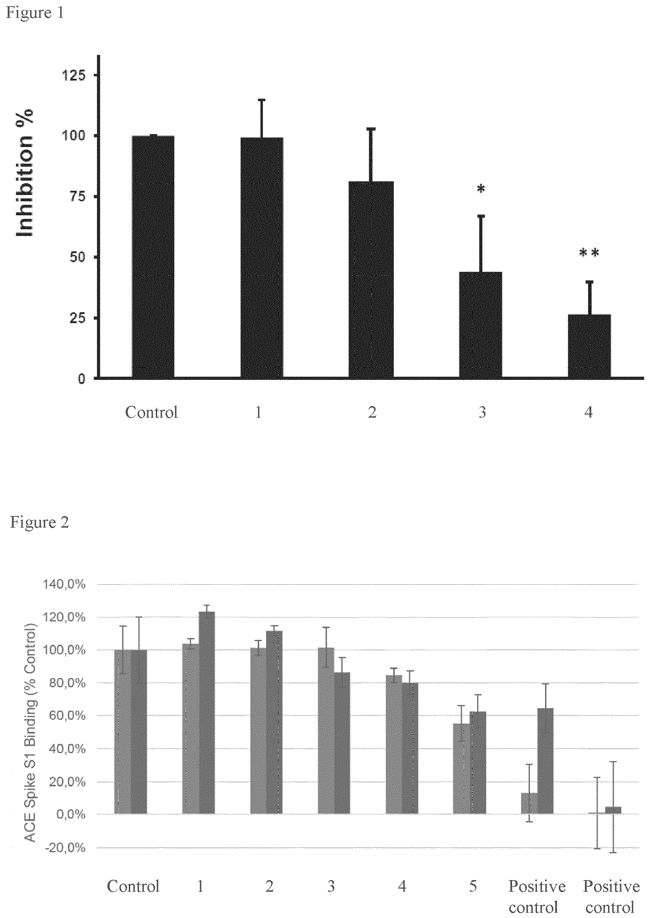

Figure 1 visualizes the effect of Composition A on HEK-Blue-ACE2 cells challenged by a SARS-CoV-2 pseudotyped virus.
   ^{∗} = p 0.05 vs control untreated cells, ^{∗∗} = p 0.01 vs control untreated cells. The infection level in the control sample, i.e. without Composition A, was set to 100%. Four samples with increasing amount of Composition A demonstrate decreased infection efficacy. The samples 1, 2, 3 and 4 contained 0.1 vol%, 1 vol%, 5 vol% and 10 vol% of Composition A, respectively.
Figure 2 visualizes the effect of Composition A on the binding of SARS-CoV-2 Spike S1 protein to ACE2 ex vitro. The control sample did not contain Composition A. Sample 1, 2, 3, 4 and 5 contained 0.01 vol%, 0.1 vol%, 1 vol%, 5 vol% and 10 vol% of Composition A, respectively. Positive controls contained 10 nM or 100 nM of an antibody known to prevent the binding of Spike S1 protein to ACE2.

### Detailed description

Coronavirus (CoV) is a family of enveloped single-stranded RNA viruses, which are infectious to animals and people. The viruses are able to cause respiratory, hepatic, enteric, and neurological diseases of various severity. At least six species of coronaviruses are known to infect humans. A group of four such coronaviruses produce symptoms that are generally mild like common cold, e.g.
Human coronavirus OC43
Human coronavirus HKU1
Human coronavirus 229E
Human coronavirus NL63

However, three human coronaviruses are known to produce potentially severe symptoms:
Severe acute respiratory syndrome coronavirus (SARS-CoV),
Middle East respiratory syndrome-related coronavirus (MERS-CoV)
Severe acute respiratory syndrome coronavirus 2 (SARS-CoV-2)

The latter three may cause the diseases commonly called SARS, MERS, and COVID-19 respectively.

It is currently believed that, as a first step of the viral replication strategy, coronaviruses attach to the host cell surface before entering the cell. The interaction between coronaviruses and host cells is believed to involve the viral Spike S1 protein and the ACE2 in the host. ACE2 is found on the surface of type I and II pneumocytes, endothelial cells, and ciliated bronchial epithelial cells. Drugs preventing the interaction between the Spike S1 protein of SARS-CoV-2 and ACE2 may thus offer some protection against the viral infection.

It is found that compositions comprising an extract from Agaricus blazei, Grifola frondosa and Hericium erinaceus inhibited SARS-CoV-2 infections of HEK-cells expressing ACE2. Accordingly, it is believed that such extracts prevent the interaction between the Spike S1 protein and ACE2.

One such composition is AndoSan^{™} which is an aqueous solution comprising a heat-sterilized extract of the of Agaricus blazei Murill, Hericeum erinaceus and Grifola frondosa. According to Hetland et al 2016 PLoS One; 11(12): e0167754, Andosan^{™} is a mixed Basidiomycetes mushroom water extract of the mycelium of (82.4%), Agaricus blazei Murill, Hericeum erinaceus (14.7%) and Grifola frondosa (2.9%). It has a dry matter content of ca 4 g per 1 and it is marketed by ImmunoPharma AS in Norway.

Several methods for culturing of Agaricus blazei are known. One example is disclosed in US5048227, and its disclosure is incorporated herein.

Methods for aqueous extraction from Agaricus blazei Murill are disclosed in US20020119164, and its disclosure is incorporated herein. The extracts in the present disclosure are solutions obtainable by subjecting fruitbody-material and/or mycelium to water, an aqueous solution, a polar non-toxic solvent and/or mixtures of these.

The correct botanical name for "Agaricus blazei" and "Agaricus blazei Murill" may be "Agaricus subrufescens". Accordingly, when used in the present disclosure, they all mean the same.

The extraction can be done by immersing mushrooms or parts thereof (e.g. mycelium, spores, cap, stipe, annulus, gill/lamellae, basidia, filaments), in any or all stages of the life cycle, whether whole or particulate (e.g. chopped or ground to powder), in water, an aqueous solution, a polar non-toxic organic solvent and/or mixtures of these. The so obtained extract is an aqueous solution if water or an aqueous solution is used for extraction. The extraction process may be performed over hours, days, weeks or months. Continuous stirring with aeration may be beneficial for the activity of the filtered composition.

Suitable temperature of the extraction-solution may be between about 25°C and 100°C.

Accordingly, suitable extracts may be obtained from the respective fruitbodies, the respective mycelium or from mixtures comprising both fruitbody-material and mycelium. Fruitbody-material may include, but is not limited to, cap-material, material from a spore-forming parts and/or stalk-material. Such extracts are preferably filtered to remove solid material.

When administration to human patients is intended, sterilization is likely needed, and it can be achieved by heat-treatment or other known sterilization methods.

The compositions in the present disclosure may thus be obtained by aqueous extraction from mixtures comprising mycelium from Agaricus blazei Murill in the range of 40 to 99 wt%, 50 to 95 wt%, 55 to 90 wt%, 60 to 85 wt% or 75 to 85 wt%. Said mixtures may also comprise mycelium from Grifola frondosa in the range of 1 to 30 wt%, 2 to 25 wt%, 5 to 20 wt % or 10 to 18 wt%. Preferably, such compositions may be mixed with aqueous extracts from Hericeum erinaceus stalks.

It is of course possible to make the suitable compositions by mixing aqueous extracts from Agaricus blazei Murill, Grifola frondosa and Hericeum erinaceus in any order.

Accordingly, such suitable compositions may for example contain a major fraction of an aqueous extract from Agaricus blazei Murill, and a minor fraction of aqueous extract from Grifola frondosa, and a minor fraction of an aqueous extracts from Hericeum erinaceus.

Accordingly, such suitable compositions may for example contain a major fraction of an aqueous extract from Agaricus blazei Murill mycelium, and a minor fraction of aqueous extract from Grifola frondosa mycelium, and a minor fraction of an aqueous extract from Hericeum erinaceus stalks.

As used herein, a major fraction means more than or equal to 50 vol%. As used herein, a minor fraction means less than 50 vol%.

All the compositions herein may comprise propolis extracts, i.e. extracts obtainable from propolis by conventional methods like maceration or Soxhlet extraction. Such method are described by Bankova et al 2021, Journal of Apicultural Research, 60:5, 734-743.

The compositions mentioned above may contain dry matter in the range of 0.1 to 100 g per 1, 0.5 to 50 g per 1, 1 to 30 g per 1, 2 to 10 g per 1 or 3 to 5 g per 1.

The pH of the compositions in the present disclosure, as conventionally measured by 25°C, may be in the range of 5 to 8, 5.5 to 7.5, 6.5 to 8 or 6 to 7.

As mentioned in US109057288, in vivo studies have demonstrated an immunological stabilizing and an anti-inflammatory effect of AndoSan^{™} both in healthy volunteers and in patients with inflammatory bowel disease when given orally. Accordingly, based on the experimental data in the present disclosure, oral administration of the compositions herein may provide an antiviral effect against coronavirus infections. However, as coronaviruses are known to infect cells in the respiratory tract, intranasal or pulmonary administration may also be effective.

Intranasal administration offers many advantages over common routes such as the oral route, as it is non-invasive and easily accessible for the administration of drugs. Further, intranasally administered compositions may be rapidly absorbed and exhibit a fast onset of action due to the rich vasculature in the submucosa. Furthermore, the avoidance of the metabolic first pass effect can lead to a higher bioavailability compared to oral administration. Intranasal administration may be achieved by suitable spraying device.

Pulmonary administration can be achieved by metered dose inhalers, nebulizers or other suitable devices.

Several metered dose inhalers are known. A metered dose inhaler is usually designed for administration and delivery of a specific dose of a pharmaceutical formulation to the lungs which upon operation by the patient, provides a short burst of aerosolized formulation, which is inhaled by the patient.

A nebulizer is a device used to administer a liquid formulation in the form of a mist which is continuously inhaled by tidal breathing. Typical administration time is 20 minutes of continuous and steady breathing through a mask. When administering a pharmaceutical composition using a nebulizer, the patients may inhale the aerosols formed by the device by tidal breathing.

It is well known that pulmonary penetration is predominantly depending on particle size and inhalation flow rate. Efficient pulmonary penetration often requires particle size in the range 1 to 5 µm. Larger particles tend to impact the oropharynx and large airways, but particles less than 1 µm tend to being exhaled. The compositions herein may be administered by inhalation of monodisperse droplets formed by aerosolization, and said droplets may for example have a mass median aerodynamic diameter of 3 to 7 µm using a suitable metered dose inhaler providing a flow of 5 to 50 L/min.

The compositions herein may be pharmaceutically acceptable aqueous solutions, capsules comprising them or lyophilized powders obtained from the pharmaceutically acceptable aqueous solutions.

"Pharmaceutically acceptable composition", as used herein, means any composition suitable and intended for in vivo use, for example administration to a patient or a subject in need thereof. As used herein, the terms "patient" and "subject" are interchangeable and refer to any human or animal individual who is receiving a composition as described herein. Such animals may include pets, farm animals and other livestock.

We thus provide a pharmaceutically acceptable composition comprising
a. an extract from Agaricus blazei
b. an extract from Grifola frondosa
c. an extract from Hericium erinaceus
for use in treatment of coronavirus infections, e.g. therapeutic or prophylactic treatment of SARS, MERS or COVID-19.

As a separate general aspect, we also provide a pharmaceutically acceptable composition comprising an aqueous extract from Agaricus blazei for use in treatment of coronavirus infections.

As a separate general aspect, we also provide a pharmaceutically acceptable composition comprising an aqueous extract from Grifola frondosa for use in treatment of coronavirus infections.

As a separate general aspect, we also provide a pharmaceutically acceptable composition comprising an aqueous extract from Hericium erinaceus for use in treatment of coronavirus infections.

As a separate general aspect, we also provide a pharmaceutically acceptable composition comprising an aqueous extract from Agaricus blazei for use in treatment of SARS-CoV-2 infections.

As a separate general aspect, we also provide a pharmaceutically acceptable composition comprising an aqueous extract from Grifola frondosa for use in treatment of SARS-CoV-2 infections.

As a separate general aspect, we also provide a pharmaceutically acceptable composition comprising an aqueous extract from Hericium erinaceus for use in treatment of SARS-CoV-2 infections.

However, the compositions herein may also be food supplements, nutraceuticals, functional food in the form of aqueous solutions, capsules comprising them or lyophilized powders obtained from the aqueous solutions. Such non-drug compositions, even if not directly indicated for treatment of coronavirus infections, may reduce the risk of getting a coronavirus infection. This effect may partly arise from prevention of the interaction between the Spike S1 protein and ACE2, but it may also partly arise from other systemic or local immune-system modulating effects. These effects may in turn reduce the coronaviruses ability to infect and/or replicate in the host. Accordingly, consuming the compositions herein, especially through long-term daily consumption, may improve the immune system readiness with respect to coronavirus challenges, such as the SARS-CoV-2 pandemic.

In the examples below, a Composition A was tested. Composition A was a solution comprising an aqueous extract from Agaricus subrufescens mycelium, and a minor fraction of aqueous extract from Grifola frondosa mycelium, and a minor fraction of an aqueous extract from Hericeum erinaceus stalks. It also contained a propolis extract as a conservative. The dry matter content was ca 4 g per 1, and the pH was around 7.

### Example 1

The effect of Composition A on the infection of cells by SARS-CoV-2 pseudotyped virus.

HEK-Blue^{™} hACE2 cells that express high levels of the human (h)ACE2 receptor at their cell surface were obtained from InvivoGen. Cells were cultured in Dulbecco's modified Eagle's medium (DMEM) supplemented with 10% (v/v) foetal bovine serum, at 37 °C in a humidified, CO2-controlled (5%) incubator.

SARS-CoV-2-pseudotyped virus stocks were produced in 293T cells by co-transfection of pNL4-3. Luc.R-E- together with pLV-SARS2-S-d19 plasmid that encodes the Spike (S) gene (codon-optimized) from the original SARS-CoV-2 Wuhan-Hu-, using the calcium phosphate transfection system. Supernatants, containing virus stocks, were harvested 48 h post-transfection and were centrifuged 5 min at 500g to remove cell debris, and stored at -80 °C until use.

It was first established that SARS-CoV-2 infected HEK-cells expressing ACE2 (HEK-Blue^{™} hACE2 cells). Other HEK-cells were far less infected. The infection was largely neutralized with a specific anti-COVID-19 monoclonal antibody.

The HEK-Blue^{™} hACE2 cells were then incubated with Composition A before the addition of the virus stocks. We found Composition A inhibited the SARS-CoV-2 pseudotyped virus from infecting HEK-Blue-ACE2.

HEK-Blue^{™} hACE2 cells (10⁵/ml) were plated on a 24-well plate and incubated with three non-cytotoxic concentrations of the test substances for 30 min. Immediately after this step the cells were inoculated with virus stocks for 1 h, then washed twice with PBS and incubated for additional 23 h. Finally, the cells were washed twice in PBS and lysed in 25 mM Tris-phosphate pH 7.8, 8 mM MgC12, 1 mM DTT, 1% Triton X-100, and 7% glycerol during 15 m at RT. Then, the lysates were spun down, and the supernatant used to measure luciferase activity using an Autolumat LB 9510 (Berthold, Bad Wildbad, Germany) following the instructions of the luciferase assay kit (Promega). The results are represented as the % of activation (considering the infected and untreated cells 100% activation) or RLU. Upon integration into host chromosomes, this recombinant virus expresses the firefly luciferase gene and consequently luciferase activity in infected cells correlates with the rate of viral replication. Thus, high luciferase activity levels were detected 24 h after cellular infection with the SARS-CoV-2 pseudotyped clone. As a positive control of the infective process a neutralizing anti-SARS-CoV-2 RBD mAb (Clone B38 from Invivogen) was used.

### Example 2

The effect of Composition A on the binding of SARS-CoV-2 Spike S1 protein to ACE2 ex vitro.

We tested the effects of Composition A in two different screenings assays analyzing the effects of test items on the interaction between ACE2 and the Spike S1 protein.

The ACE2:SARS-CoV-2 Spike S1 Inhibitor Screening Assay Kit is designed for screening and profiling inhibitors of this interaction.

As visualized in Figure 2 and the table below, Composition A shows inhibitory effects in duplicate assays measuring the interaction between ACE2 and Spike S1 protein.

| **Control** | **0.01 vol% Composition A** | **0.1 vol% Composition A** | **1 vol% Composition A** | **5 vol% Composition A** | **10 vol% Composition A** | **10 nM antibody** | **100 nM antibody** |
|---|---|---|---|---|---|---|---|
| 100,0 % | 103.9 % | 101.3 % | 101.5 % | 84.6 % | 55.3 % | 13.1 % | 1.0 % |
| 100,0 % | 123.3 % | 111.5 % | 86.3 % | 80.1 % | 62.6 % | 64.3 % | 4.5 % |

## Claims

1. A pharmaceutically acceptable composition comprising
a. an extract from Agaricus blazei,
b. an extract from Grifola frondosa, and/or
c. an extract from Hericium erinaceus
for use in treatment of coronavirus infections.

2. A pharmaceutically acceptable composition according to claim 1, wherein the composition comprises
an extract from Agaricus blazei,
an extract from Grifola frondosa, and
an extract from Hericium erinaceus.

3. A pharmaceutically acceptable composition according to claim 1 or 2, wherein the treatment is therapeutic or prophylactic.

4. A pharmaceutically acceptable composition according to any one of claim 1 to 3, wherein the coronavirus is SARS-CoV-2.

5. A pharmaceutically acceptable composition according to any one of claim 1 to 4, wherein the composition is an aqueous solution.

6. A pharmaceutically acceptable composition according to any one of claim 1 to 5, wherein the composition is administered orally, intranasally or pulmonary.

7. A pharmaceutically acceptable composition according to any one of claim 1 to 6, wherein the composition comprises an extract obtained by aqueous extraction of a mixture comprising 50 to 90 wt% mycelium from Agaricus blazei.

8. A pharmaceutically acceptable composition according to any one of claim 1 to 7, wherein the composition comprises an extract obtained by aqueous extraction of a mixture comprising 10 to 50 wt% mycelium from Grifola frondosa.

9. A pharmaceutically acceptable composition according to any one of claim 1 to 8, wherein the composition comprises an extract obtained by aqueous extraction from Hericium erinaceus stalks.

10. A pharmaceutically acceptable composition according to any one of claim 1 to 9, wherein the extracts are obtained by aqueous extraction of mycelium from Agaricus blazei, mycelium from Grifola frondosa and Hericium erinaceus stalks.

11. A pharmaceutically acceptable composition according to any one of claim 1 to 10, wherein the extract from Agaricus blazei is an aqueous solution comprising 2 g to 3.6 g dry matter material per liter.

12. A pharmaceutically acceptable composition according to any one of claim 1 to 11, wherein the extract from Grifola frondosa is an aqueous solution comprising 0.04 g to 0.8 g dry matter material per liter.

13. A pharmaceutically acceptable composition according to any one of claim 1 to 12, wherein the extract from Hericium erinaceus is an aqueous solution comprising 0.4 g to 1.2 g dry matter material per liter.

14. A pharmaceutically acceptable composition according to any one of claim 1 to 13, wherein the pH is the range of 5 to 8.
